(19) Europäisches Patentamt
European Patent Office
Office européen des brevets

(11) **EP 1 245 242 B1**

(12) **EUROPÄISCHE PATENTSCHRIFT**

(45) Veröffentlichungstag und Bekanntmachung des Hinweises auf die Patenterteilung:
**30.12.2015  Patentblatt 2015/53**

(51) Int Cl.:
***A61M 1/36*** *(2006.01)*

(21) Anmeldenummer: **02003083.9**

(22) Anmeldetag: **13.02.2002**

(54) **Verfahren und Vorrichtung zum Erkennen von Stenosen in einem Schlauchleitungssystem**

Method and apparatus for the detection of a stenosis in a system tube

Méthode et appareil de détection de sténose dans un tube du système

(84) Benannte Vertragsstaaten:
**AT BE CH CY DE DK ES FI FR GB GR IE IT LI LU MC NL PT SE TR**

(30) Priorität: **30.03.2001  DE 10115991**

(43) Veröffentlichungstag der Anmeldung:
**02.10.2002  Patentblatt 2002/40**

(73) Patentinhaber: **Fresenius Medical Care Deutschland GmbH**
**61352 Bad Homburg v.d.H. (DE)**

(72) Erfinder: **Gross, Malte, Dr.**
**89075 Ulm (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**EP-A- 0 995 451       EP-A- 1 044 695**
**WO-A-97/10013       DE-C- 19 901 078**
**US-A- 4 969 470**

**Beschreibung**

Die Erfindung betrifft ein Verfahren und eine Vorrichtung zum Erkennen von Engstellen in einem Schlauchleitungssystem während einer extrakorporalen Blutbehandlung.

Die extrakorporale Blutbehandlung ist heute ein Standardverfahren, das vor allem zur Behandlung der Niereninsuffizienz in Form der Hämodialyse, Hämofiltration oder Hämodiafiltration eingesetzt wird. Zur extrakorporalen Blutbehandlung ist ein ausreichend ergiebiger Zugang zum Blutkreislauf des Patienten erforderlich. Als Blutzugang hat sich seit vielen Jahren ein unter der Haut liegender Shunt zwischen einer Arterie und Vene bewährt. Während der extrakorporalen Blutbehandlung strömt das Blut des Patienten über den arteriellen Zweig des Schlauchleitungssystems in die Blutbehandlungseinheit, beispielsweise einen Hämodialysator oder Hämofilter, und strömt von der Blutbehandlungseinheit über den venösen Zweig des Leitungssystems zurück zum Patienten. Das Blut wird mittels einer volumetrischen Blutpumpe, insbesondere Rollenpumpe gefördert, die im arteriellen Zweig des Leitungssystems angeordnet ist. Die bekannten Schutzsysteme überwachen im allgemeinen den Druck des Bluts sowohl im arteriellen Zweig als auch im venösen Zweig des Leitungssystems. Hierfür ist ein arterieller Drucksensor stromauf der Blutpumpe und ein venöser Drucksensor stromab der Blutbehandlungseinheit vorgesehen.

In der Praxis kommt es gelegentlich vor, daß der Blutschlauch zwischen arterieller Blutpumpe und Dialysator abknickt. Eine besonders gefährdete Stelle liegt unmittelbar vor dem Eingang des Dialysators. Das Abknicken führt zu einer Engstelle (Stenose) im extrakorporalen Kreislauf, an der sich eine große Druckdifferenz aufbaut. Das Blut wird mit hoher Geschwindigkeit durch die

Stenose hindurch gepreßt. Die extrem großen Geschwindigkeitsgradienten können zu Scherkräften führen, denen die Erythrozyten nicht mehr widerstehen; Hämolyse ist die Folge. Bleibt ein derartiges Abknicken längere Zeit unbemerkt, kann die Hämolyse zu lebensbedrohenden Gesundheitsschäden führen.

Zwar wird ein vollständiger Verschluß des Blutschlauchs durch den venösen Drucksensor erkannt, die vorhandenen Schutzsysteme können jedoch eine noch teilweise durchgängige Engstelle nur bedingt detektieren. Eine Stenose vor der Blutbehandlungseinheit führt zwar zu einer Erhöhung des Drucks am Ausgang der Blutpumpe, die Förderleistung der letzteren bleibt jedoch in einem weiten Rahmen nahezu konstant. Dadurch ändern sich die Druckwerte weder am arteriellen Drucksensor, noch am venösen Drucksensor signifikant. Letztere hängen im wesentlichen nur von der Förderrate und den Flußwiderständen im Patientenzugang bzw. -rücklauf ab.

Eine sicherere Methode zum Erkennen einer Knickstelle im Leitungssystem wäre das Einfügen eines zusätzlichen Drucksensors am Ausgang der arteriellen Blutpumpe. Diese Lösung ware jedoch mit einem erheblichen Mehraufwand seitens der Schlauchsysteme verbunden. Zusätzliche Anschlußstücke mit Hydrophobfiltern etc. wären erforderlich.

Die DE-A-199 01 078 beschreibt eine Vorrichtung zur Erkennung von Stenosen bei der extrakorporalen Blutbehandlung, die von dem arteriellen Drucksensor Gebrauch macht. Die Vorrichtung beruht darauf, daß die Blutpumpe ein oszillierendes Drucksignal generiert, das sich über das Schlauchleitungssystem fortpflanzt. Bei einer hinreichend starken Stenose zwischen Blutpumpe und Blutbehandlungseinheit steigt der Druck nach der Blutpumpe so stark an, daß die Okklusion der Pumpenrollen teilweise aufgehoben wird. Dies hat zur Folge, daß beim Pumpvorgang zeitweise etwas Blut in das Leitungssystem vor die Blutpumpe zurückströmt, was zu einer Vergrößerung der am arteriellen Drucksensor gemessenen Pulsamplitude führt. Gleichzeitig bewegt sich der im allgemeinen negative arterielle Mitteldruck in Richtung Nullinie, da die Fördermenge nachläßt.

Das obige Verfahren ist in der Praxis jedoch nur bedingt tauglich, eine beginnende Stenose zu erkennen, da die Okklusion der Pumpe erst bei einem Förderdruck von ca. 2 bar nachläßt. Bis dahin ist am arteriellen Drucksensor keine signifikante Änderung des Drucksignals zu beobachten. Dennoch können die hohen Druckgradienten an der Stenose bereits zu einer gefährlichen Hämolyse fuhren.

Aus der WO 97/10013 ist ein Verfahren zur Erkennung einer Stenose am Patientenzugang bekannt, bei dem ein oszillierendes Drucksignal analysiert wird, das über das Schlauchleitungssystem übertragen wird. Aus dem Drucksignal wird das auf die Rotation der Blutpumpe zurückzuführende Pumpensignal extrahiert, um das Pulssignal des Patienten detektieren zu können, das zur Erkennung der Stenose herangezogen wird.

Der Erfindung liegt daher die Aufgabe zugrunde, ein bei den bekannten Blutbehandlungseinrichtungen einfach zu realisierendes Verfahren zum Erkennen von Engstellen in einem Schlauchleitungssystem anzugeben, das sich durch eine hohe Empfindlichkeit auszeichnet, so daß die Erkennung von Engstellen bereits möglich ist, bevor Veränderungen in Mittelwert und Amplitude von arteriellem und venösem Drucksignal erkennbar werden. Darüber hinaus ist eine Aufgabe der Erfindung, eine Vorrichtung zum Erkennen von Engstellen in einem Schlauchleitungssystem bereitzustellen.

Die Lösung dieser Aufgabe erfolgt erfindungsgemäß mit den Merkmalen der Patentansprüche 1 bzw. 7.

Das erfindungsgemäße Verfahren und die erfindungsgemäße Vorrichtung nutzen die Änderung des dynamischen Verhaltens des Schlauchleitungssystems bei einer Engstelle (Stenose) aus. Ursache für die Änderung des dynamischen Verhaltens ist die Compliance des Leitungssystems, d. h. das elastische Nachgeben unter Druck.

Diese Compliance wird nicht als Störfaktor angesehen, sondern gezielt zur Stenosendetektion ausgenutzt.

Auf der Grundlage der Änderung des dynamischen Verhaltens kann bereits eine beginnende Stenose sicher erkannt werden, bevor die Okklusion der arteriellen Blutpumpe aufgehoben wird. Es kann von den Schlauchleitungssystemen (Disposables) der bekannten Blutbehandlungseinrichtungen Gebrauch gemacht werden, ohne daß zusätzliche Sensoren oder dgl. erforderlich sind.

Das erfindungsgemäße Verfahren sieht eine Analyse des Frequenzspektrums des oszillierenden Drucksignals vor. Auf eine Stenose wird bei einer Änderung des Frequenzspektrums geschlossen, die auf eine Änderung des dynamischen Verhaltens des Schlauchleitungssystems aufgrund der Verengung zurückzuführen ist.

Für das erfindungsgemäße Verfahren ist unerheblich, wie das oszillierende Drucksignal in dem Schlauchleitungssystem generiert wird. Vorzugsweise werden die Druckpuise der volumetrischen Blutpumpe, insbesondere Rollenpumpe als oszillierendes Signal gemessen, mit der das Blut im arteriellen Zweig gefördert wird.

Die Änderung des dynamischen Verhaltens des Schlauchleitungssystems hat eine unterschiedliche Dämpfung des oszillierenden Drucksignals zur Folge. Eine Stenose führt zu einer starken Dämpfung insbesondere bei höheren Frequenzen.

Es wird eine Analyse, vorzugsweise Fouriertransformation, des oszillierenden Drucksignals durchgeführt und die Dämpfung mindestens einer Oberschwingung des Drucksignals ermittelt. Aus der Änderung der Dämpfung wird dann auf eine Stenose geschlossen. Eine Stenose kann sicher erkannt werden, wenn nur die Dämpfung der ersten Oberschwingung ermittelt wird. Es kann aber auch die Dämpfung einer oder mehrerer Oberschwingungen einer höheren Ordnung als die erste Oberschwingung ermittelt werden. Prinzipiell ist es möglich, auf eine Stenose dann zu schließen, wenn eine Dämpfung bezüglich nur einer Oberschwingung oder eine Dämpfung mehrerer Oberschwingungen vorliegt. Dabei können bei der Auswertung die bekannten statistischen Verfahren herangezogen werden, um die Empfindlichkeit oder Sicherheit weiter zu erhöhen.

Im folgenden werden das Verfahren und die Vorrichtung unter Bezugnahme auf die Zeichnungen anhand von Beispielen näher erläutert.

Es zeigen:

Fig. 1    eine schematische Darstellung eines extrakorporalen Blutkreislaufs,

Fig. 2    ein elektrisches Ersatzschaltbild des extrakorporalen Blutkreislaufs von Fig. 1,

Fig. 3    die Fouriertransformierte des venösen oszillierenden Drucksignals, wobei eine Stenose nicht vorliegt,

Fig. 4    die Fouriertransformierte des oszillierenden Drucksignals, wobei eine Stenose vorliegt,

Fig. 5    eine schematische Darstellung der wesentlichen Komponenten einer Vorrichtung zum Erkennen von Stenosen und

Fig. 6    eine weitere Ausführungsform der Vorrichtung zum Erkennen von Stenosen.

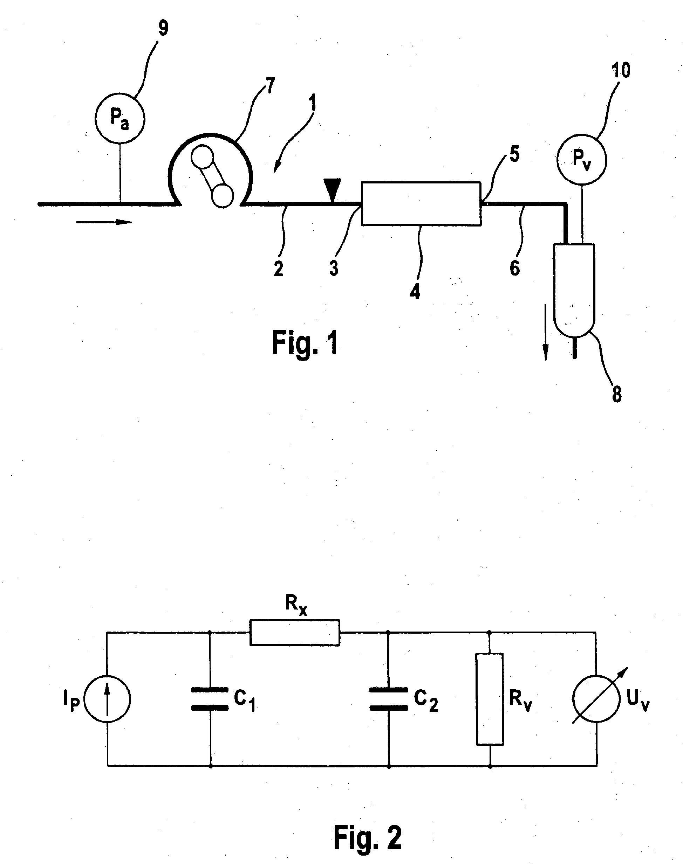

Figur 1 zeigt den extrakorporalen Blutkreislauf in schematischer Darstellung. Das Blut des Patienten strömt durch den arteriellen Zweig 1 eines als Disposable ausgebildeten Schlauchleitungssystems 2 zu dem Eingang 3 einer Blutbehandlungseinheit 4, beispielsweise eines Dialysators. Von dem Ausgang 5 des Dialysators 4 strömt das Blut durch den venösen Zweig 6 des Leitungssystems 2 zurück zum Patienten. Das Blut wird mit einer volumetrischen Blutpumpe, insbesondere Rollenpumpe 7 gefördert, die stromauf des Dialysators 4 in den arteriellen Zweig 1 des Leitungssystems 2 geschaltet ist. In den venösen Zweig 6 des Leitungssystems ist eine Tropfkammer 8 geschaltet. Der Blutdruck im arteriellen Zweig 1 stromauf der Blutpumpe 7 wird mit einem arteriellen Drucksensor 9 und der Druck im venösen Zweig 6 stromab des Dialysators mit einem venösen Drucksensor 10 überwacht. Der Dialysator 4, das Schlauchleitungssystem 2, die Blutpumpe 7, die Tropfkammer 8 sowie die Drucksensoren 9, 10 sind Bestandteil der bekannten Blutbehandlungseinrichtungen, beispielsweise Hämodialysevorrichtungen.

Während der extrakorporalen Blutbehandlung pflanzen sich die Druckwellen der Blutpumpe 7 als oszillierendes Drucksignal über das Schlauchleitungssystem 2 fort. Figur 2 zeigt ein elektrisches Ersatzschaltbild, mit dem die hydrodymanischen Eigenschaften des Leitungssystems beschrieben werden können.

Die Blutpumpe entspricht einer Stromquelle mit dem eingeprägten Strom $I_P$, der am venösen Drucksensor 10 gemessene Druck der Spannung $U_V$. Die durch das Abquetschen entstehende Engstelle wird durch den Widerstand $R_X$ der Abflußwiderstand am Patientenzugang, der im wesentlichen durch die venöse Nadel hervorgerufen wird, durch $R_V$ dargestellt. $C_1$ und $C_2$ stehen für die Compliance, d. h. die elastische Dehnbarkeit des Blutsystems vor bzw. nach der Engstelle. $C_1$ wird durch das Schlauchsystem zwischen arterieller Pumpe sowie der nicht dargestellten arteriellen Tropfkammer, die zwischen Pumpe 7 und Dialysator angeordnet ist, und der Engstelle bestimmt; $C_2$ wird bestimmt durch das Schlauchsystem nach der Engstelle, den Dialysator 4 und die venöse Tropfkammer 8. Eine Analyse der komplexen Übertragungsfunktion (im Frequenzraum) des obigen Netzwerkes liefert für ein sinusförmiges Eingangssignal $I_P$ mit Kreisfrequenz $\omega$:

$$U_V = I_P \frac{R_V}{1 - \omega^2 R_X R_V C_1 C_2 + j\omega(R_X C_1 + R_V(C_1 + C_2))}$$

Gleichung 1

$I_P$ ist ein periodisches Signal, dessen Periodendauer der halben Umdrehungszeit eines Blutpumpenrotors entspricht, der über zwei symmetrisch angeordnete Rollen verfügt. Man kann daher ansetzen:

$$I_P = I_{P0} + I_{P1}e^{j\omega_P t} + I_{P2}e^{2j\omega_P t} + I_{P3}e^{3j\omega_P t} + \ldots$$

Dabei ist $I_{P0}$ der kontinuierliche Anteil des Pumpenflusses, $\omega_P$ die Grundfrequenz der Blutpumpe (doppelte Rotationsfrequenz). $I_{P1}$, $I_{P2}$, $I_{P3,\ldots}$ sind die (komplexen) Amplituden von Grund- und Oberwellen. Aufgrund der Linearität des Netzwerkes gilt dann für das Ausgangssignal:

$$U_V = U_{V0} + U_{V1}e^{j\omega_P t} + U_{V2}e^{2j\omega_P t} + U_{V3}e^{3j\omega_P t} + \ldots$$

Für die Beträge der Amplituden gilt:

$$U_{V0} = I_{P0}R_V$$

$$|U_{V1}| = |I_{P1}| \frac{R_V}{\sqrt{\left(1 - \omega_P^2 R_X R_V C_1 C_2\right)^2 + \omega_P^2 (R_X C_1 + R_V(C_1 + C_2))^2}}$$

$$|U_{V2}| = |I_{P2}| \frac{R_V}{\sqrt{\left(1 - 4\omega_P^2 R_X R_V C_1 C_2\right)^2 + 4\omega_P^2 (R_X C_1 + R_V(C_1 + C_2))^2}}$$

Gleichung 2

usw.

Wie schon zuvor erwähnt, ist der statische Anteil $D_{V0}$ vom Widerstand der Engstelle $R_X$ unabhängig. Für höhere Frequenzen wirkt das Netzwerk als Tiefpaßfilter 2. Ordnung. Es werden nun zwei Spezialfälle betrachtet: $R_X=0$ und $R_V<<R_X$.

Im Fall $R_X=0$, d. h. ohne Stenose im Schlauchsystem, folgt aus Gleichung 1:

$$U_V = I_P \frac{R_V}{1 - j\omega R_V(C_1 + C_2)}$$

Gleichung 3

Dies ist die Übertragungsfunktion eines Tiefpasses 1. Ordnung mit der Grenzfrequenz $\omega_g=1/(R_V(C_1+C_2))$. Es hat sich im Laborversuch gezeigt, daß die Oberwellen der Blutpumpe bis etwa zur 3. Ordnung durch diesen Tießpaß nicht sichtbar gedämpft werden, solange das Schlauchsystem keine Stenose enthält. Das heißt, daß die Grenzfrequenz des Tiefpasses deutlich oberhalb von $3\omega_P$ liegen muß. Es gilt also:

$$\omega_P R_V(C_1 + C_2) << 1$$

Das bedeutet, daß man in erster Näherung die Terme $\omega_P R_V C_1$ und $\omega_P R_V C_2$ vernachlässigen kann. Damit erhält man für den Fall $R_V<<R_X$:

$$U_V = I_P \frac{R_V}{1 - j\omega R_X C_1}$$

Gleichung 4

Bei zunehmend stärker werdenden Abknicken verhält sich das Blutsystem daher wie ein Tiefpaß mit Grenzfrequenz $\omega_g = 1/(R_X C_1)$. Je stärker der Schlauch abgeknickt wird, desto tiefer sinkt die Grenzfrequenz, bis sie unter die Blutpumpengrundfrequenz fällt. Die höheren Harmonischen der Blutpumpe werden noch stärker gedämpft als die Grundschwingung. Nur noch die Grundschwingung ist sichtbar. Wenn man den Betrag der Signalamplituden betrachtet, folgt aus Gleichung 4:

$$|U_V| = |I_P| \frac{R_V}{\sqrt{1 - \omega^2 R_X^2 C_1^2}}$$

$R_V$ kann aus dem statischen Drucksignal bestimmt werden (Gleichung 2). Die Frequenz $\omega$ der Blutpumpe bzw. deren Harmonische sind aus dem Spektrum bekannt. Daher ist es möglich, $R_X C_1$ zu bestimmen, so daß eine Größe vorliegt, die der Stärke der Quetschung des Schlauches direkt proportional ist. $C_1$ ist die Compliance des Blutsystems zwischen Blutpumpe und Engstelle und hängt von deren Lage sowie von Geometrie und Material des Schlauches ab. $C_1$ ist um so größer, je weiter der Knick von der Blutpumpe entfernt ist. Die Methode ist also besonders empfindlich auf Knicke in der Nähe des Dialysators.

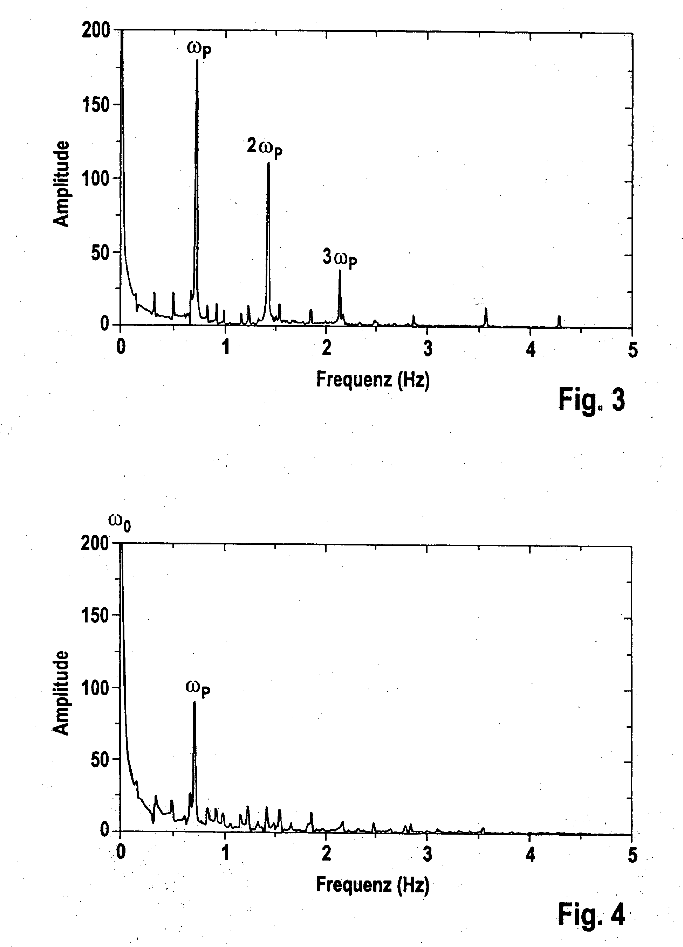

Nachfolgend wird das Verfahren zum Erkennen von Stenosen beschrieben. Das oszillierende Drucksignal im venösen Zweig 6 des Schlauchleitungssystems 2 wird mit dem venösen Drucksensor 10 gemessen. Anschließend wird eine Fouriertransformation des oszillierenden Drucksignals durchgeführt. Figur 3 zeigt die Fouriertransformierte des venösen Drucksignals mit der Grundschwirigung $\omega_P$ sowie den Oberschwingungen $2\omega_P$, $3\omega_P$, ... vor dem Abquetschen des arteriellen Zweigs 1 des Leitungssystems 2. Die Fouriertransformierte des Drucksignals nach dem Abquetschen des arteriellen Zweigs 1 zwischen Blutpumpe 7 und Dialysator 4 zeigt Figur 4. Die Stenose ist in Figur 1 mit einem Pfeil gekennzeichnet. Deutlich ist zu erkennen, daß die Amplituden insbesondere der Oberschwingungen $2\omega_P$, $3w\omega_P$, ... kleiner sind. Die erste Oberschwingung $2\omega_P$ und alle Oberschwingungen höherer Ordnungen sind kaum noch zu erkennen. Damit kann aus der Dämpfung der Oberschwingungen sicher auf eine Stenose geschlossen werden, selbst dann, wenn die Grundschwingung nicht gedämpft werden sollte, was sich in Versuchen gezeigt hat. Dabei kann das Abknicken des Schlauchs von der Erhöhung des Strömungswiderstandes am Patientenzugang, wie er z. B. durch den Anstieg der Blutviskosität entstehen kann, eindeutig unterschieden werden. Neben einer Stenose kann mit dem Verfahren auch das Verstopfen des Dialysators sicher erkannt werden. Insofern kann das Verfahren auch zur Überwachung des Dialysators eingesetzt werden.

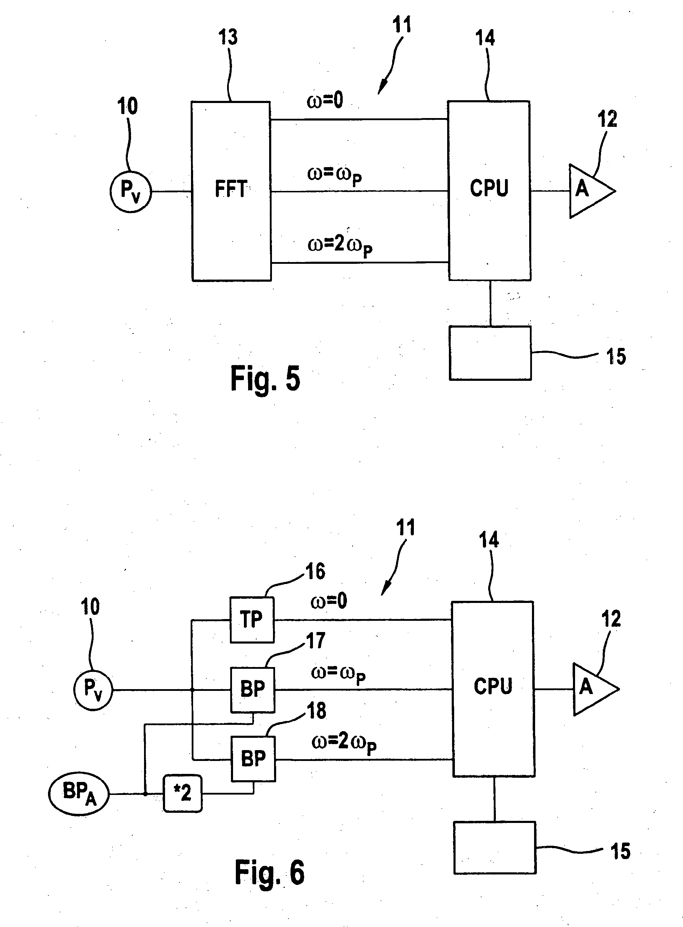

Figur 5 zeigt ein erstes Ausführungsbeispiel der Vorrichtung zum Erkennen von Stenosen, die eine separate Baugruppe oder Bestandteil der bekannten Blutbehandlungseinrichtungen sein kann.

Die Vorrichtung weist einen venöse Drucksensor 10 zum Messen des oszillierenden Drucksignals im venösen Zweig 6 des Schlauchleitungssystems 2 des extrakorporalen Blutkreislaufs der Blutbehandlungseinrichtung auf. Der venöse Drucksensor 10 ist im allgemeinen Bestandteil der bekannten Blutbehandlungseinrichtungen (Figur 1). Darüber hinaus weist die Vorrichtung Mittel 11 zum Analysieren des mit dem Drucksensor 10 gemessenen Drucksignals und eine Alarmeinrichtung 12 auf. Die Mittel 11 zum Analysieren des Drucksignals umfassen Mittel 13 zum Durchführen einer Fouriertransformation FFT und eine zentrale Recheneinheit 14 CPU, die das Frequenzspektrum des Drucksignals analysiert. Darüber hinaus ist eine Speichereinheit 15 vorgesehen.

Aus dem Fourierspektrum des Drucksignals werden der statische Anteil ($\omega=0$), die Blutpumpengrundfrequenz ($\omega_P$) und die erste Oberschwingung ($\omega=2\omega_P$) extrahiert. Zu Beginn der Blutbehandlung wird die Amplitude der ersten Oberschwingung in die Speichereinheit 15 als Referenzwert eingelesen. Dabei wird davon ausgegangen, daß eine Stenose nicht vorliegt. Alternativ kann aber auch ein in Vergleichsversuchen ermittelter Referenzwert für die erste Oberschwingung fest vorgegeben sein. Während der Blutbehandlung wird die erste Oberschwingung aus dem Fourierspektrum kontinuierlich extrahiert. Die CPU 14 weist Mittel zum Bilden einer Differenz zwischen dem Referenzwert und dem extrahierten Wert der Amplitude auf. Die Differenz wird mit einem vorgegebenen Grenzwert verglichen. Ist die Differenz größer als der Grenzwert, so gibt die CPU 14 ein Alarmsignal an die Alarmeinrichtung 12. Die Alarmeinrichtung 12 gibt nun einen optischen und/oder akustischen Alarm, da eine Stenose vorliegt.

Zur Erhöhung der Sicherheit und Empfindlichkeit kann die Analyse des Frequenzspektrums auch auf der Grundlage von weiteren Oberschwingungen einer höheren Ordnung erfolgen. In der Speichereinheit 15 ist dann für jede Oberschwingung ein Referenzwert gespeichert. Die CPU 14 bildet die Differenz zwischen jedem Referenzwert und der zugehörigen Amplitude der Oberschwingung. Die CPU 14 erzeugt das Alarmsignal dann, wenn für wenigstens eine Oberschwingung die Differenz größer als ein vorgegebener Grenzwert ist.

Figur 6 zeigt ein weiteres Ausführungsbeispiel der Vorrichtung zum Erkennen von Stenosen, die sich von der Ausführungsform gemäß Figur 5 durch die Mittel zum Durchführen der Fouriertransformation unterscheidet. Die einander entsprechenden Teile sind mit den gleichen Bezugszeichen versehen.

Die Ausführungsform von Figur 6 weist zum Extrahieren von dem statischen Anteil, der Blutpumpengrundfrequenz und der erster Oberschwingung einen Tiefpaß 16 sowie zwei frequenzvariable Bandpässe 17, 18 auf, die jeweils einen Lock-In-Verstärker bilden. Der Tiefpaß 16 filtert das zeitgemittelte Signal ($\omega$=0) heraus. Als Frequenzbasis für den ersten Bandpaß 17 wird das arterielle Blutpumpensignal $BP_A$ verwendet, während das mit dem Faktor 2 multiplizierte Blutpumpensignal als Frequenzbasis für den zweiten Bandpaß 18 verwendet wird. Das arterielle Blutpumpensignal $BP_A$ ist ein mit der Drehzahl der Blutpumpe korrelierendes Signal. Bei einer Rollenpumpe mit zwei Rollen beispielsweise hat das Blutpumpensignal die doppelte Frequenz im Vergleich zu der Drehbewegung des Pumpenrotors. Dieses Signal kann beispielsweise an der Steuerung der Pumpe oder einem separaten Drehzahlmesser abgegriffen werden.

Der erste Bandpaß 17 extrahiert die Blutpumpengrundfrequenz und der zweite Bandpaß 18 die erste Oberschwingung. Ansonsten sind die Vorrichtungen gemäß der Figuren 5 und 6 baugleich.

**Patentansprüche**

1. Verfahren zum Erkennen von Engstellen in einem Schlauchleitungssystem während einer extrakorporalen Blutbehandlung, das einen vom Patienten abgehenden und zu einer Blutbehandlungseinheit führenden arteriellen Zweig und einen von der Blutbehandlungseinheit abgehenden und zu dem Patienten führenden venösen Zweig aufweist, bei dem ein oszillierendes Drucksignal in dem Schlauchleitungssystem generiert und das oszillierende Drucksignal gemessen und analysiert wird, **dadurch gekennzeichnet, daß** das Frequenzspektrum des oszillierenden Drucksignals analysiert und die Dämpfung mindestens einer Oberschwingung des Drucksignals ermittelt wird und aus der Änderung der Dämpfung auf eine Engstelle geschlossen wird.

2. Verfahren nach Anspruch 1, **dadurch gekennzeichnet, daß** die Dämpfung der ersten Oberschwingung und/oder einer oder mehrerer Oberschwingungen einer höheren Ordnung als die erste Oberschwingung ermittelt wird.

3. Verfahren nach Anspruch 1 oder 2, **dadurch gekennzeichnet, daß** zur Ermittlung einer Oberschwingung des oszillierenden Drucksignals eine Fouriertransformation des oszillierenden Drucksignals durchgeführt wird.

4. Verfahren nach einem der Ansprüche 1 bis 3, **dadurch gekennzeichnet, daß** die Differenz der zu Beginn der Behandlung ermittelten Amplitude einer Oberschwingung und der während der Behandlung ermittelten Amplitude einer Oberschwingung des oszillierenden Drucksignals berechnet wird, wobei auf eine Stenose dann geschlossen wird, wenn die Differenz größer als ein vorgegebener Grenzwert ist.

5. Verfahren nach einem der Ansprüche 1 bis 4, **dadurch gekennzeichnet, daß** das Blut in dem arteriellen Zweig mit einer volumetrischen Blutpumpe, insbesondere Rollenpumpe, gefördert wird, deren Druckpulse als oszillierendes Drucksignal gemessen werden.

6. Verfahren nach einem der Ansprüche 1 bis 5, **dadurch gekennzeichnet, daß** das oszillierende Drucksignal mit einem Drucksensor gemessen wird, der in dem venösen Zweig des Schlauchleitungssystems stromab der Blutbehandlungseinheit angeordnet ist.

7. Vorrichtung zum Erkennen von Engstellen in einem Schlauchleitungssystem (2) während einer extrakorporalen Blutbehandlung, das einen vom Patienten abgehenden und zu einer Blutbehandlungseinheit (4) führenden arteriellen Zweig (2) und einen von der Blutbehandlungseinheit abgehenden und zu dem Patienten führenden venösen Zweig (6) aufweist, mit

Mitteln (7) zum Generieren eines oszillierenden Drucksignals in dem Schlauchleitungssystem,
Mitteln (10) zum Messen des oszillierenden Drucksignals, und
Mitteln (11) zum Analysieren des oszillierenden Drucksignals,
**dadurch gekennzeichnet, daß**
die Mittel zum Analysieren des oszillierenden Drucksignals Mittel (13;16,17,18;14) zum Analysieren des Frequenzspektrums des Drucksignals und Ermitteln der Dämpfung mindestens einer Oberschwingung des oszillierenden Drucksignals aufweisen, die aus der Änderung der Dämpfung auf eine Engstelle schließen.

**8.** Vorrichtung nach Anspruch 7, **dadurch gekennzeichnet, daß** die Mittel (13;16,17,18;14) zum Analysieren des oszillierenden Drucksignals Mittel zum Ermitteln der Dämpfung der ersten Oberschwingung und/oder einer oder mehrerer Oberschwingungen einer höheren Ordnung als die Oberschwingung aufweisen.

**9.** Vorrichtung nach Anspruch 7 oder 8, **dadurch gekennzeichnet, daß** die Mittel (13;16,17,18;14) zum Analysieren des oszillierenden Drucksignals Mittel zum Durchführen einer Fouriertransformation (13) aufweisen.

**10.** Vorrichtung nach einem der Ansprüche 7 bis 9, **dadurch gekennzeichnet, daß** die Mittel (13;16,17,18;14) zum Analysieren des oszillierenden Signals Mittel zum Bilden der Differenz der zu Beginn der Behandlung ermittelten Amplitude einer Oberschwingung und der während der Behandlung ermittelten Amplitude einer Oberschwingung des oszillierenden Drucksignals aufweisen, die auf eine Stenose dann schließen, wenn die Differenz größer als ein vorgegebener Grenzwert ist.

**11.** Vorrichtung nach einem der Ansprüche 8 bis 10, **dadurch gekennzeichnet, daß** die Mittel zum Generieren des oszillierenden Drucksignals eine volumetrische Blutpumpe (7), insbesondere Rollenpumpe sind.

**12.** Vorrichtung nach einem der Ansprüche 8 bis 11, **dadurch gekennzeichnet, daß** die Mittel zum Messen des oszillierenden Drucksignals einen venösen Drucksensor (10) umfassen, der stromab der Blutbehandlungseinheit (4) angeordnet ist.

**Claims**

**1.** Method for detecting stenoses in a hose line system during extracorporeal blood treatment, said hose line system comprising an arterial segment leading away from the patient and leading to a blood treatment unit, and a venous segment leading away from the blood treatment unit and leading to the patient, during which method an oscillating pressure signal is generated in the hose line system and said oscillating pressure signal is measured and analysed, **characterised in that** the frequency spectrum of the oscillating pressure signal is analysed, the attenuation of at least one harmonic component of the pressure signal is determined, and the presence of a stenosis is concluded from the change in the attenuation.

**2.** Method according to claim 1, **characterised in that** the attenuation of the first harmonic component and/or of one or more harmonic components having a higher order than the first harmonic component is determined.

**3.** Method according to either claim 1 or claim 2, **characterised in that** a Fourier transform of the oscillating pressure signal is carried out in order to determine a harmonic component of the oscillating pressure signal.

**4.** Method according to any of claims 1 to 3, **characterised in that** the difference between the amplitude of a harmonic component of the oscillating pressure signal determined at the beginning of the treatment and the amplitude of a harmonic component of the oscillating pressure signal determined during the treatment is calculated, the presence of a stenosis being concluded if the difference is greater than a predetermined threshold value.

**5.** Method according to any of claims 1 to 4, **characterised in that** the blood in the arterial segment is conveyed by means of a volumetric blood pump, in particular a roller pump, the pressure pulse of which is measured as the oscillating pressure signal.

**6.** Method according to any of claims 1 to 5, **characterised in that** the oscillating pressure signal is measured by means of a pressure sensor which is arranged in the venous segment of the hose line system, downstream of the blood treatment unit.

**7.** Device for detecting stenoses in a hose line system (2) during extracorporeal blood treatment, said hose line system comprising an arterial segment (2) leading away from the patient and leading to a blood treatment unit (4), and a venous segment (6) leading away from the blood treatment unit and leading to the patient, comprising
means (7) for generating an oscillating pressure signal in the hose line system,
means (10) for measuring the oscillating pressure signal, and
means (11) for analysing the oscillating pressure signal,
**characterised in that**
the means for analysing the oscillating pressure signal comprise means (13; 16, 17, 18; 14) for analysing the frequency spectrum of the pressure signal and for determining the attenuation of at least one harmonic component of the oscillating pressure signal, which means conclude the presence of a stenosis from the change in the attenuation.

8. Device according to claim 7, **characterised in that** the means (13; 16, 17, 18; 14) for analysing the oscillating pressure signal comprise means for determining the attenuation of the first harmonic component and/or of one or more harmonic components having a higher order than the harmonic component.

9. Device according to either claim 7 or claim 8, **characterised in that** the means (13; 16, 17, 18; 14) for analysing the oscillating pressure signal comprise means for carrying out a Fourier transform (13).

10. Device according to any of claims 7 to 9, **characterised in that** the means (13; 16, 17, 18; 14) for analysing the oscillating signal comprise means for establishing the difference between the amplitude of a harmonic component of the oscillating pressure signal determined at the beginning of the treatment and the amplitude of a harmonic component of the oscillating pressure signal determined during the treatment, which means then conclude the presence of a stenosis if the difference greater than a predetermined threshold value being.

11. Device according to any of claims 8 to 10, **characterised in that** the means for generating the oscillating pressure signal are a volumetric blood pump (7), in particular a roller pump.

12. Device according to any of claims 8 to 11, **characterised in that** the means for measuring the oscillating pressure signal comprise a venous pressure sensor (10) which is arranged downstream of the blood treatment unit (4).

**Revendications**

1. Procédé permettant de détecter des rétrécissements dans un système de tuyau flexible pendant un traitement extracorporel du sang, qui comporte une voie artérielle partant du patient et menant vers une unité de traitement du sang et une voie veineuse partant de l'unité de traitement du sang et menant vers le patient, dans lequel procédé un signal de pression oscillant est généré dans le système de tuyau flexible et le signal de pression oscillant est mesuré et analysé, **caractérisé en ce que** le spectre de fréquences du signal de pression oscillant est analysé et l'affaiblissement d'une composante harmonique du signal de pression est déterminé, et la présence d'un rétrécissement est déduite à partir de la variation de l'affaiblissement.

2. Procédé selon la revendication 1, **caractérisé en ce que** l'affaiblissement de la première composante harmonique et/ou d'une ou de plusieurs composantes harmoniques d'un ordre supérieur à la première composante harmonique est déterminée.

3. Procédé selon la revendication 1 ou 2, **caractérisé en ce qu'**une transformation de Fourier du signal de pression oscillant est effectuée pour déterminer une composante harmonique du signal de pression oscillant.

4. Procédé selon l'une quelconque des revendications 1 à 3, **caractérisé en ce que** la différence entre l'amplitude déterminée au début du traitement pour une composante harmonique et l'amplitude déterminée pendant le traitement pour une composante harmonique du signal de pression oscillant est calculée, si la différence est supérieure à une valeur limite prédéfinie, on en déduit alors la présence d'une sténose.

5. Procédé selon l'une quelconque des revendications 1 à 4, **caractérisé en ce que** le sang est acheminé vers la voie artérielle au moyen d'une pompe à sang volumétrique, en particulier une pompe à rouleaux, dont les impulsions de pression sont mesurées en tant que signal de pression oscillant.

6. Procédé selon l'une quelconque des revendications 1 à 5, **caractérisé en ce que** le signal de pression oscillant est mesuré au moyen d'un capteur de pression qui est disposé dans la voie veineuse du système de tuyau flexible en aval de l'unité de traitement du sang.

7. Dispositif permettant de détecter des rétrécissements dans un système de tuyau flexible (2) pendant un traitement extracorporel du sang, qui comporte une voie artérielle (2) partant du patient et menant vers une unité de traitement du sang (4) et une voie veineuse (6) partant de l'unité de traitement du sang et menant vers le patient, lequel dispositif comporte :

des moyens (7) pour générer un signal de pression oscillant dans le système de tuyau flexible,
des moyens (10) pour mesurer le signal de pression oscillant, et
des moyens (11) pour analyser le signal de pression oscillant,
**caractérisé en ce que**
les moyens pour analyser le signal de pression oscillant comportent des moyens (13 ; 16, 17, 18 ; 14) pour analyser le spectre de fréquences du signal de pression oscillant et déterminer l'affaiblissement d'au moins une composante harmonique du signal de pression oscillant, lesquels moyens, à partir de la variation de l'affaiblissement, déduisent la présence d'un rétrécissement.

8. Dispositif selon la revendication 7, **caractérisé en ce que** les moyens (13 ; 16, 17, 18 ; 14) pour analyser le spectre de fréquences du signal de pression oscillant comportent des moyens pour déterminer l'affaiblissement de la première composante harmonique et/ou d'une ou de plusieurs composantes harmoniques d'un ordre supérieur à la première composante harmonique.

9. Dispositif selon la revendication 7 ou 8, **caractérisé en ce que** les moyens (13 ; 16, 17, 18 ; 14) pour analyser le spectre de fréquences du signal de pression oscillant comportent des moyens pour effectuer une transformation de Fourier (13).

10. Dispositif selon l'une quelconque des revendications 7 à 9, **caractérisé en ce que** les moyens (13 ; 16, 17, 18 ; 14) pour analyser le spectre de fréquences du signal de pression oscillant comportent des moyens pour former la différence entre l'amplitude déterminée au début du traitement pour une composante harmonique et l'amplitude déterminée pendant le traitement pour une composante harmonique du signal de pression oscillant, lesquels moyens, lorsque la différence est supérieure à une valeur limite prédéfinie, en déduisent alors la présence d'une sténose.

11. Dispositif selon l'une quelconque des revendications 8 à 10, **caractérisé en ce que** les moyens pour générer le signal de pression oscillant sont une pompe à sang (7) volumétrique, en particulier une pompe à rouleaux.

12. Dispositif selon l'une quelconque des revendications 8 à 11, **caractérisé en ce que** les moyens pour mesurer le signal de pression oscillant comportent un capteur de pression veineuse (10) qui est disposé en aval de l'unité de traitement du sang (4).

9

7

1

10

P_a

5

P_v

2

3

4

6

8

**Fig. 1**

$R_x$

$I_P$

$C_1$

$C_2$

$R_v$

$U_v$

**Fig. 2**

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19901078 A

- WO 9710013 A